# EUROPEAN PATENT APPLICATION

(11) **EP 4 700 782 A1**
(43) Date of publication of application: **25.02.2026**
(21) Application number: 24195531.9
(22) Date of filing: 21.08.2024
(51) Int. Cl.: G16H 15/00, G16H 30/40

(54) **SYSTEM AND METHOD FOR CONTROLLING PERCEPTIBILITY OF A MEDICAL VISUALIZATION**

(71) Applicant: Koninklijke Philips N.V., 5656 AG Eindhoven (NL)
(72) Inventor: TESSEL, Uli, 5656 AG Eindhoven (NL); BUIL, Vincentius Paulus, 5656 AG Eindhoven (NL)
(74) Representative: Philips Intellectual Property & Standards

(57) **Abstract**

Some embodiments are directed to a system and a method for controlling perceptibility of a medical visualization, wherein physiological data of a patient is mapped to a medical visualization, wherein the mapping is defined by visualization parameters comprised in a visual encoding scheme. A perceptibility of a visualization parameter of the visual encoding scheme is compared to a reference perceptibility value, wherein the reference perceptibility value indicates a level of perceptibility to a human perception system of a user. Based on a decision if one or more visualization parameters of the visual encoding scheme are to be modified, perceptibility of the medical visualization is controlled by modifying the one or more visualization parameters of the visual encoding scheme, the modifying causing the rendering part to adjust the medical visualization accordingly.

## Description

### FIELD OF THE INVENTION

The presently disclosed subject matter relates to a system for controlling perceptibility of a medical visualization. The presently disclosed subject matter further relates to a method of controlling perceptibility of a medical visualization. The presently disclosed subject matter further relates to a transitory or non-transitory computer-readable medium comprising data representing a computer program, wherein the computer program comprises instructions for causing a processor system to perform the method of controlling perceptibility of a medical visualization.

### BACKGROUND OF THE INVENTION

In health-care settings, such as hospital wards, operating rooms (ORs), intensive care units (ICUs), and ambulances, extensive use is made of systems and devices which present patient monitoring data. Such systems and devices are also used in other medical settings, such as home products for use at home or in a retirement home, such as health-monitoring applications on smart devices and thereon and other consumer health products. As in recent times the amount and potential complexity of available patient data has become larger, the computers processing patient data have become increasingly powerful, and the available display monitor screens have become larger and are able to show more complex visualizations, the importance and potential of patient data monitoring systems is widely recognized. With the modern available monitoring systems and devices, a plurality of parameters may be displayed and visualized: e.g., a heart rate, oxygen saturation, blood pressure, respiratory rates and expiratory carbon dioxide. Such parameters may be displayed in numerical form, and/or in graphical form, such as a wave, e.g. periodic waves, data points, e.g. in point clouds. Such visualization may also be a dynamic visualization, i.e., an animation, so changing over time.

There exist many systems for monitoring the medical state of a patient through a dynamically rendered model, which may be presented through a medical visualization. An example of such a medical visualization is a patient monitor rendering a Visual Patient Avatar (VPA): see, e.g., the published European patent EP3311315. A VPA may comprise an avatar which may be physiologically guided, to enable a health-care provider, such as a doctor, an ICU nurse, and/or an OR anesthetist, to discern and distinguish the visualizations of parameters in the plurality of parameters from each other in a better view. This way, the overview may be improved for a user of the visualization system, as a large number of physiological data from a patient may be presented, and it may be difficult to remain timely aware of the physiological situation and state of the patient. EP3311315 may address the problem of maintaining a consistent overview and comprehension of the time-varying information on the physiological state of the patient and remain aware of the medical situation: this property is called "situation awareness of the user", wherein the user may be the healthcare provider.

Systems as the one in EP3311315, comprising an avatar, e.g. patient monitors comprising a VPA, may display physiological patient data in a physiologically guided avatar. The VPA may use a visual coding scheme that shows a logical connection between measured physiological parameters and the visualization. The visualization may comprise an animation, e.g., a visualization in which visualization parameters, such as color, shape, size, transparency, and/or orientation, change over time. The animation may loop, in which case it may repeat at a certain rate. A rate may be used to visualize a certain physiological state of a patient: e.g., a slow rate may be used to express something has a low value. For example, a heart rate (HR) may be visualized via a pulsing animation of a heart shape. A high HR may be visualized as fast pulses, e.g., a short loop with a fast-repeating rate. A low HR may be visualized as slow pulses, e.g., a long loop with a slow-repeating rate. An avatar of a heart may indicate the HR; likewise, a lung avatar may indicate inhalation and exhalation. Over time, the avatar visualizations may change in, e.g., size and/or color in a synchronous manner to the time-varying parameters, to indicate a heart rate via heart pumping and/or respiration rate via air inhalation and air exhalation. This relationship may be quick and easy to understand and comprehend for a user of the system. This way, a VPA aims to improve situation awareness about a patient for the user, e.g. a healthcare provider as an OR anesthetist or an ICU nurse, such that the user may more quickly notice via their perception system, i.e., in peripheral view, and may more quickly understand via their comprehension system whether they should act on the medical situation and the state of the patient, if it is declining.

### SUMMARY OF THE INVENTION

A technical problem of the prior art may be, that the visual coding of measured physiological parameters may conflict with the human perception system of the user and lead to a decline in situation awareness of the user in medical situations in which the user, e.g. a health-care provider, should provide medical aid, and/or a declining physiological state of a patient which should be noticed by the user may be visualized with a low perceptibility to the human perception system. Such medical situations lead to a counter-productive functioning of the prior-art visualization systems in combination with the human perception system, as such visualizations are disadvantageous for the overview: users may not know in time what to concentrate and/or to focus on, nor act accordingly.

For example, a dangerously low heart rate may be visualized in an animation of a heart avatar using a slow animation frequency. Slow movements are typically not perceived by the human eye and brain, especially if only noticed by peripheral vision, by which such medical situations which need attention typically go unnoticed by the user due to the nature of the human perception system. Other coding mechanisms may go unnoticed by the human perceptive system in similar ways, such as small size differences in animations visualizing expiratory carbon dioxide and subtle color changes visualizing oxygen saturation drops, especially with multiple things happening simultaneously in dynamic medical visualizations. This way, it may become difficult to the human visual perception system to distinguish between the different visualization parameters in the visualization and notice medical situations which ask for the user's attention.

It would be advantageous to have an improved system in which perceptibility and therefore noticeability of medical situations to the human perception of a user of a medical visualization system is increased in peripheral vision.

In accordance with a first aspect of the invention, a system is provided for controlling perceptibility of a medical visualization, wherein the system comprises a processor system and a memory storing instructions that, when executed by the processor system, cause the processor system to perform operations for:
- a rendering part, wherein the rendering part maps physiological data of a patient to a medical visualization, wherein the mapping is defined by visualization parameters comprised in a visual encoding scheme,
- comparing a perceptibility of a visualization parameter of the visual encoding scheme to a reference perceptibility value, wherein the reference perceptibility value indicates a level of perceptibility to a human perception system of a user,
- based on the comparing, deciding if one or more visualization parameters of the visual encoding scheme are to be modified, and
- based on the deciding, controlling perceptibility of the medical visualization by modifying the one or more visualization parameters of the visual encoding scheme, the modifying causing the rendering part to adjust the medical visualization accordingly.

In accordance with a further aspect of the invention, a method of controlling perceptibility of a medical visualization is provided, comprising:
- mapping physiological data of a patient to a medical visualization, wherein the mapping is defined by visualization parameters comprised in a visual encoding scheme,
- comparing a perceptibility of a visualization parameter of the visual encoding scheme to a reference perceptibility value, wherein the reference perceptibility value indicates a level of perceptibility to a human perception system of a user,
- based on the comparing, deciding if one or more visualization parameters of the visual encoding scheme are to be modified, and
- based on the deciding, controlling perceptibility of the medical visualization by modifying the one or more visualization parameters of the visual encoding scheme.

In accordance with a further aspect of the invention, a transitory or non-transitory computer-readable medium is provided comprising data representing a computer program, the computer program comprising instructions for causing a processor system to perform any of the methods described in this specification.

The above measures involve that the perceptibility of the medical visualization may be controlled independently of the visual encoding scheme. By independently controlling the perceptibility, a visualization system according to the invention may be enabled to make a certain medical situation perceptible, and/or more perceptible, and thereby noticeable and/or more noticeable to a user of the system, or less perceptible, and thereby less noticeable to a user of the system, independently of whether and how the perceptibility would be coded, mapped, and presented to a user by the visual encoding scheme. This way, the mapping of physiological data, defined by the visualization parameters, may be decoupled from the perceptibility of the visualization parameters.

A further aspect of the invention is, that the modifying may comprise modifying the visualization parameter of which the perceptibility is compared to the reference perceptibility value, to make it more perceptible and therefore noticeable, or less perceptible and noticeable, to a user of the system. The visualization parameter may be modified in its value, based on the comparison to the reference perceptibility value but independently of the mapping defined by the visual encoding scheme, to modify its perceptibility and noticeability.

Optionally, the modifying comprises modifying one or more visualization parameters which are different from the visualization parameter of which the perceptibility is compared to the reference perceptibility value. The visualization parameters may comprise one or more of color, shape, size, transparency, brightness, orientation, contrast, gradient, glow, fluency of an animation in the medical visualization, and a repetition frequency of the medical visualization. This way, to compensate for a perceptibility of a first visualization parameter, e.g., the repetition frequency of vital signs in an animation, for which it is clear from the comparing that there is a risk that the corresponding medical situation is not noticeable to a user, e.g., in the case of a too low frequency of the vital signs to go noticed by a user, by which the information on the vital signs are ignored, the perceptibility of a second visualization parameter, e.g., the size of the animation, may be modified in order to increase the likelihood of the noticeability of the medical situation and avoid the potential error of missing the important information.

Optionally, the system is further configured, based on the comparing, to add a visual and/or an auditory signal to the medical visualization, such as a symbol and/or a sound. A symbol, a sound, other visual and/or auditory signals, and/or other types of signals may be added. Such signals may be independent from the visualization parameters. Adding such signals may particularly be advantageous in the cases in which a user may not perceive or notice a medical situation in the visualization which should be noticed at all, e.g., in a case in which the user does not pay attention to the medical visualization at all. An alarm symbol and/or an alarming auditory signal may then bring back the user's attention to the medical visualization.

Optionally, the system if further configured to select the visualization parameter of which the perceptibility is compared. This selection may be based on data the system may comprise and/or access, such as values of the physiological data of the patient, rendering the process more patient-specific. Optionally, the system may be configured to enable a user to select the visualization parameter of which the perceptibility is compared. The user-selectability of the visualization parameter may render the process more user-friendly and/or user-specific (for example, in case of color blindness of the user). Optionally, the system may further comprise a database. The database may comprise data, such as the reference perceptibility values. The reference perceptibility values may be user-specific and/or clinic-specific. The reference perceptibility values may be based on research on the human perception system, such as, e.g., Tscholl et al. (2018) Using an Animated Patient Avatar to Improve Perception of Vital Sign Information by Anaesthesia Professionals, Tscholl et al. (2020) The Mechanisms Responsible for Improved Information Transfer in Avatar-Based Patient Monitoring, Pfarr et al. (2019) Avatar-Based Patient Monitoring with Peripheral Vision*,* and Kawahara et al. (2012) Attentional capture by the onset and offset of motion signals outside the spatial focus of attention*.* Such a database as part of the system may render the process more patient-specific, user-specific and/or clinic-specific.

The method of controlling perceptibility of a medical visualization as described above may be implemented on a device, such as a device used in the medical applications. It may be an electronic device, in particular a computer. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer. It may be able to carry out the methods mentioned above. It may be a mobile electronic device, e.g., a smart phone. On a computer, the methods may be implemented as new software, or a new software feature of some existing software application utilized for segmentation tasks, like the applications that are found in systems as in Intelispace Portal, DynaCAD or Multimodality Simulator. Executable code for an embodiment of the method may be stored on a computer program product. Examples of computer program products include memory devices, optical storage devices, integrated circuits, servers, online software, etc. Preferably, the computer program product comprises non-transitory program code stored on a computer-readable medium for performing an embodiment of the method when said program product is executed on a computer.

An embodiment provides a computer-readable medium allowing to perform the methods above. On this medium, the embodied computer program comprises computer program code adapted to perform all or part of the steps of an embodiment of the method when the computer program is run on a computer. Preferably, the computer program is embodied on a computer readable medium.

Another aspect of the presently disclosed subject matter is a method of making the corresponding computer program available for downloading, or sharing via, e.g., a cloud storage. This aspect is used when the computer program is uploaded into, e.g., Apple's App Store, Google's Play Store, or Microsoft's Windows Store, and when the computer program is available for downloading from such a store.

The method of controlling perceptibility of a medical visualization as described above may be applied in a wide range of practical and/or medical applications. Such applications comprise systems and devices which present patient monitoring data, such as patient data monitoring systems, as used in health-care settings such as hospital wards, operating rooms, intensive care units, ambulances, etc., and other medical settings, such as home products for use at home or in a retirement home, such as health-monitoring applications on smart devices and thereon and other consumer health products. Such devices are generally electronic devices, such as a computer, and/or smart mobile electronic devices, such as a smart mobile phone, and/or a smart card. The home products generally comprise consumer electronics, such as a set-top box, a television. The systems and devices in the applications generally involve displays such as monitor screens for presenting patient data. Such applications may comprise any patient data monitoring system, or any other practical and/or medical applications which make use of any platform displaying patient data. Philips products like the Philips IntelliVue MX750 bedside patient monitor, any other Philips IntelliVue patient monitors, and any other Philips product using the Visual Patient Avatar are examples of such applications. Furthermore, in other industries than medical imaging similar situations a similar method may be of use for monitoring situations and/or processes for which the attention of a user, like a technician, a supervisor or an process expert, is divided over several components and/or subprocesses.

A person skilled in the art will appreciate that the method may be applied to multidimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

The embodiments as mentioned above are described in the accompanying claims. Further, specific embodiments are set forth in the dependent claims.

### BRIEF DESCRIPTION OF THE DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Fig. 1 schematically shows an example of an embodiment of a system for controlling perceptibility of a medical visualization,
Fig. 2 schematically shows an example of a foveal view versus a peripheral view in a system according to an example of an embodiment,
Fig. 3 schematically shows an example of a flow of controlling perceptibility of a medical visualization according to an example of an embodiment,
Figs. 4a and 4b schematically show examples of medical visualizations wherein perceptibility is controlled according to an example of an embodiment,
Fig. 5 schematically shows a medical visualization of which perceptibility is controlled according to an example of an embodiment,
Figs. 6a, 6b, 6c and 6d schematically show examples of medical visualizations of which perceptibility is controlled according to an example of an embodiment,
Fig. 7 schematically shows an example of an embodiment of a method of controlling perceptibility of a medical visualization,
Fig. 8a schematically shows a computer readable medium having a writable part comprising a computer program according to an embodiment,
Fig. 8b schematically shows a representation of a processor system according to an embodiment.

### Reference signs list

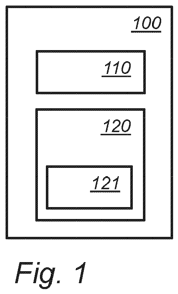

The following list of references and abbreviations corresponds to Figs. 1-8b, and is provided for facilitating the interpretation of the drawings and shall not be construed as limiting the claims.
- 100: a system for controlling perceptibility of a medical visualization
- 110: processor system
- 120: memory
- 121: database

- 201: patient
- 202: head
- 203: focus
- 204: situation awareness
- 205: user
- 210: foveal view
- 220: peripheral view

- 300: flow of controlling perceptibility of a medical visualization
- 310: sensor
- 321: physiological data
- 330: rendering part
- 341: visualization parameters
- 350: visual encoding scheme
- 361: medical visualization
- 371: reference perceptibility values
- 380: modifying
- 400: medical visualizations
- 401-3, 411-3: visual representations
- 421: repetition rates
- 422: time
- 423: apparent size

- 500: visual patient avatar
- 501, 502, 503: stages of apparent size of a medical visualization of a heart
- 511, 512, 513: stages of apparent size of a medical visualization of an exhaling
- 520: ice flake symbol

- 601-3, 611-3, 621-3: medical visualization of a heart

- 700: method of controlling perceptibility of a medical visualization
- 710: obtaining physiological data of a patient
- 720: mapping the physiological data to a medical visualization
- 730: selecting a visualization parameter of which the perceptibility is to be compared
- 740: comparing the perceptibility of the visualization parameter to a reference perceptibility value
- 750: deciding if one or more visualization parameters are to be modified
- 760: controlling the perceptibility of the medical visualization by modifying the one or more visualization parameters

- 770: adding a visual and/or an auditory signal to the medical visualization

- 1000, 1001: a computer-readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

**Fig. 1** schematically shows an example of an embodiment of a system 100 for controlling perceptibility of a medical visualization. System 100 may be part of a medical visualization system, such as a patient monitor.

System 100 is configured to control the perceptibility of a medical visualization. The medical visualization may be obtained by mapping physiological data. The physiological data may be physiological data of a patient. The patient may, e.g., be a heart patient, and the physiological data may, e.g., represent heart rates. The mapping using which the physiological data is mapped to the medical visualization may be defined by visualization parameters. The visualization parameters may be comprised in a visual encoding scheme. The visualization parameters may comprise one or more of: color, shape, size, transparency, brightness, orientation, contrast, gradient, glow, fluency of an animation in the medical visualization, and a repetition frequency of the medical visualization. The visualization parameters may also comprise a difference between visualization parameters, a variation in time of a visualization parameter, and/or a derivative or slope of a visualization parameter. The mapping of the physiological data to the medical visualization may be carried out by a rendering part. The rendering part may be part of system 100. The rendering part may be external to system 100. System 100 may communicate with the rendering part via a communication surface. System 100 may be able to monitor the medical visualization via communicating with the rendering part. System 100 may monitor the medical visualization by communicating with the rendering part that he medical visualization may be adjusted. System 100 may control perceptibility of the medical visualization by modifying one or more visualization parameters of the visual encoding scheme which define the mapping rendering part 330 carries out. The rendering part may be part of a system, e.g., a medical visualization system of which system 100 is also a part. The rendering part may be a rendering part of, e.g., a patient monitor, of which system 100 is also a part. The rendering part may render the medical visualization of the medical visualization system system 100 is a part of.

For example, system 100 may be used to be applied in the medical imaging field. Applications may comprise systems and devices which present patient monitoring data, such as patient monitors. Such applications may comprise patient data visualized in medical visualizations, e.g., in a visual patient avatar; such as Philips products like the Philips IntelliVue MX750 bedside patient monitor. In such applications, perceptibility of the medical visualizations which are used for monitoring a patient may need to be controlled of it is found that the noticeability of certain medical situations and/or the situation awareness of users of the systems in such applications may need to be increased.

System 100 may comprise a processor system 110 and a memory 120. Memory 120 may store instructions that, when executed by processor system 110, cause processor system 110 to perform operations for executing a method. Optionally, system 100 may comprise a communication interface, e.g., to communicate with a rendering part. Memory 120 may comprise a storage, e.g., electronic storage, magnetic storage, etc. The storage may comprise local storage, e.g., a local hard drive or electronic memory. The storage may comprise non-local storage, e.g., cloud storage. In the latter case, the storage may comprise a storage interface to the non-local storage. The storage may comprise multiple discrete sub-storages together making up memory 120. The storage may comprise non-transitory storage. For example, the storage may store data in the presence of power such as a volatile memory device, e.g., a Random Access Memory (RAM). For example, memory 120 may store data in the presence of power as well as outside the presence of power such as a non-volatile memory device, e.g., Flash memory. Memory 120 may comprise a non-volatile non-writable part, e.g., ROM, e.g., storing part of the software.

System 100 may communicate internally, e.g. with an internal rendering part, with other systems, e.g., with an external rendering part, with other devices, external storage, input devices, output devices, and/or one or more sensors over a computer network. The computer network may be an internet, an intranet, a LAN, a WLAN, etc. The computer network may be the Internet. The computer network may be wholly or partly wired, and/or wholly or partly wireless. System 100 may comprise a connection interface which is arranged to communicate within system 100 or outside of system 100 as needed. For example, the connection interface may comprise a connector, e.g., a wired connector, e.g., an Ethernet connector, an optical connector, etc., or a wireless connector, e.g., an antenna, e.g., a Wi-Fi, ZigBee, a cellular antenna, a 4G or 5G antenna. System 100 may communicate via a communication interface, which is configured to communicate with other devices or systems. The communication interface may be selected from various alternatives. For example, the interface may be a network interface to a local or wide area network, e.g., the Internet, a storage interface to an internal or external data storage, an application programming interface (API), etc. The network may be a computer network. The network may comprise additional elements, e.g., a router, a hub, etc. The communication interface may be used to send or receive data, e.g., physiological data of a patient. System 100 may be connected to a cloud storage system, to which multiple systems such as system 100 may be connected as well. This may illustrate an option of uploading, storing, sharing and downloading data which may be obtained from modifications of medical visualizations, e.g., by multiple systems, users or clinics. This data may describe user-specific or clinic-specific behavior, characterized, e.g., by the modifications of the medical visualizations which are displayed. In this computing cloud implementation, each user or clinic may contribute data, reflecting their own behavior. It may allow users or clinic to obtain insight in perceptibility of medical visualizations and noticeability of medical situations. This data may therefore be meaningful.

System 100 may further be configured to select visualization parameters of which the perceptibility may be compared. System 100 may be configured to select visualization parameters based on physiological data of a patient, e.g., based on the data values.

System 100 may further comprise a display, e.g., a monitor screen. The medical visualization may be displayed on the display. The display may further be configured to visualize visual signals, such as symbols, e.g., alarming symbols. The visual signals may not be part of the medical visualization. System 100 may further comprise an audio output. The audio output may be configured to output an auditory signal, such as a sound, e.g., an alarming sound. The auditory need not be part of the medical visualization. System 100 may further comprise a sensor. System 100 may be configured to obtain physiological data of a patient from the sensor. System 100 may comprise a database 121. Database 121 may be comprised in memory 120. Database 121 may comprise data, e.g., physiological data, e.g., physiological data of a patient, and/or of a clinic. The physiological data may be patient-specific, and/or clinic-specific, and/or user-specific. The physiological data may be specific to a healthcare type. The data in database 121 may comprise reference perceptibility values. The reference perceptibility value may be clinic-specific and/or user-specific.

System 100 may comprise a user interface, which may include well-known elements such as one or more buttons, a keyboard, display, touch screen, etc. The user interface may be arranged for accommodating user interaction for performing, e.g., selections of visualization parameters, e.g., visualization parameters of which perceptibility may be compared to a reference perceptibility value. A user may be able to select visualization parameters based on physiological data of a patient, e.g., based on the data values. System 100 may be configured to enable the user to select the visualization parameter of which the perceptibility is compared. System 100 may, for example, be configured to present the physiological data of the patient, e.g., on a display.

The execution of system 100 may be implemented in a processor system. System 100 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. System 100 may comprise multiple processors. A processor circuit may be implemented in a distributed fashion, e.g., as multiple sub-processor circuits. For example, system 100 may use cloud computing.

System 100 may comprise a microprocessor which executes appropriate software stored at the device; for example, that software may have been downloaded and/or stored in a corresponding memory, e.g., a volatile memory such as RAM or a non-volatile memory such as Flash.

Instead of using software to implement a function, system 100 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, system 100 may comprise circuits, e.g., for cryptographic processing, and/or arithmetic processing.

In hybrid embodiments, functional units are implemented partially in hardware, e.g., as coprocessors, and partially in software stored and executed on the device.

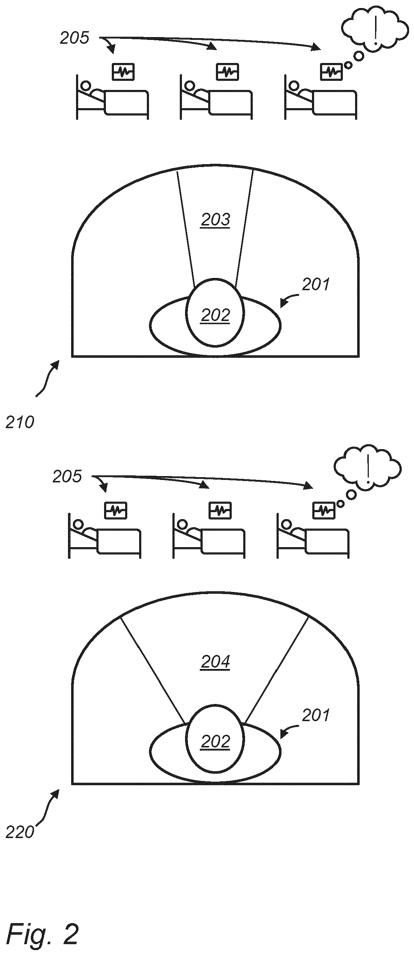

**Fig. 2** schematically shows an example of a foveal view 210 versus a peripheral view 220 in a system 100 according to an example of an embodiment. A user 201 is shown from a top view, with his head 202 looking forward. The foveal view, 210, with as a corresponding region the focus 203, encompasses a small region in the total visual field of the user 201 of system 100. User 201 may be a doctor, a surgeon, an OR anesthetist, an ICU nurse, or any other healthcare provider. He may be observing a number of patients 205 via multiple patient monitors in real life, or via a nurse station overview showing multiple patients on one computer screen. As user 201 as a healthcare provider typically has to concentrate on a main specific medical situation for which the user is attended to, user 201 may be inclined to a focus view 203, covering only the middle patient in the diagram. The human perception system of user 201 tends to work that way: see also experimental research discussed in, e.g., Pfarr et al. (2019) Avatar-Based Patient Monitoring with Peripheral Vision, and Kawahara et al. (2012) Attentional capture by the onset and offset of motion signals outside the spatial focus of attention*.* Focus 203, however, takes up only a small region of the full view user 201 may have in total. The peripheral region 220, however, encompasses a larger portion of the full visual field user 201 would have if they would have a total overview. With this total overview, user 201 would have situation awareness 204. However, the human perception is known to be prone to focus and concentrate, and therefore be limited to the focus view 203; especially the healthcare provider as user 201. Either way, a full peripheral view 204 generally is not covered by the view of user 201. Therefore, to arrive at full situation awareness 204, system 100 may assist user 201 in reaching such a state of situation awareness. To arrive at full situation awareness 204, first a certain level of perception must be reached, in which all necessary data and elements of the environment and medical situation of all patients 205 are perceived. Above this state of perception, there is a level of comprehension which needs to be reached, in which the meaning and significance of the medical situation needs to be understood. After this state of comprehension, there is a third state of projection, in which future states and events may be understood from the situation. In this final state, encapsulating the two previous states, one may speak of situation awareness 204. As user 201 generally may not be able to cover the region of peripheral vision, a larger and better view may be created by system 100. With system 100, user 201 may be enabled to discern and distinguish the visualization parameters in the larger, better view. This way, the overview may be improved for user 201, as a large number of physiological data from a patient may be presented, and it may be more easily facilitated to remain timely aware of the medical situation and state of the patients. With improving the overview for user 201, controllable noticeability of situation awareness presentations in medical visualizations 361 may be achieved, in which it is allowed to control the perception of medical visualizations in peripheral vision 220, to get or lose attraction of users 201.

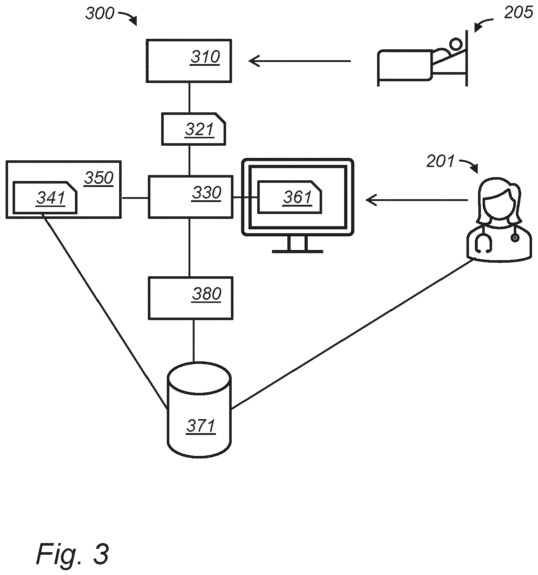

**Fig. 3** schematically shows an example of a flow 300 of controlling perceptibility of a medical visualization 361 according to an example of an embodiment. First, physiological data 321 of a patient 205 may be obtained. Physiological data 321 may be obtained using a sensor 310. Sensor 310 may be comprised in system 100. Sensor 310 may be external to system 100. System 100 may obtain physiological data 321 from an external sensor. System 100 may obtain physiological data 321 from a communication interface of system 100. The communication interface may be connected to sensor 310. A rendering part 330 may map physiological data 321 of patient 205 to a medical visualization 361. Rendering part 330 may be comprised in system 100. Rendering part 330 may be external to system 100. System 100 may obtain medical visualization 361 from an external rendering part 330. System 100 may obtain medical visualization 361 from a communication interface of system 100. The communication interface may be connected to rendering part 330. Medical visualization 361 may be obtained as a visual representation of one or more bodily functions of patient 205, such as a heart rate, blood pressure and/or respiration, e.g., as a visual patient avatar. Medical visualization may be formed on a display, such as a monitor screen. The display may be external to system 100. System 100 may communicate medical visualization 361 to the display. System 100 may communicate medical visualization 361 via a communication interface of system 100. The mapping of rendering part 330 may be defined by visualization parameters 341. Visualization parameters 341 may be comprised in a visual encoding scheme 350. Visual encoding scheme 350 may encapsulate the way physiological data 321 data generally is mapped into the visual structures which together form a visual representation of medical visualization 361; in other words, how the final image, medical visualization 361, is built and formed on a display, e.g., a monitor screen. Visual encoding scheme 350 may comprise one or more visualization patterns. The one or more visualization patterns may comprise visualization parameters 341. A visualization pattern may comprise two or more visualization parameters 341. The two or more visualization parameters 341 may be jointly adaptable. The visualization parameters 341 may be jointly adaptable in the sense, that they may be modified together: if one of the two or more visualization parameters, e.g. a size, may be modified, a second of the two or more visualization parameters, e.g. a color palette, may be modified along with the modification of the first visualization parameter. Visual encoding scheme 350 may serve an easily comprehensible coding. An easily comprehensible coding may, partly or as a whole, may be formed by a logical visual mapping of visualization parameters 341 to bodily functions, such as a heart rate, blood pressure and/or respiration. Visualization parameters 341 may comprise one or more of: color, shape, size, transparency, brightness, orientation, contrast, gradient, glow, fluency of an animation in the medical visualization, and a repetition frequency of the medical visualization. Visualization parameters 341 may also comprise a difference between visualization parameters, a variation in time of a visualization parameter, and/or a derivative or slope of a visualization parameter. A perceptibility of a visualization parameter 341 of visual encoding scheme 350 may be compared to a reference perceptibility value 371 The perceptibility of visualization parameter 341 may be compared by system 100. Reference perceptibility value 371 may be comprised in database 121. Database 121 may be comprised in system 100, e.g., in memory 120. Database 121 may be external to system 100. System 100 may obtain reference perceptibility value 371 from an external database 121. System 100 may obtain reference perceptibility value 371 from a communication interface of system 100. The communication interface may be connected to database 121. Reference perceptibility value 371 may be comprised in system 100 otherwise, e.g., in memory 120. Reference perceptibility value 371 may indicate a level of perceptibility of visualization parameter 341. Reference perceptibility value 371 may indicate a level of perceptibility of visualization parameter 341 to a human perception system, e.g., of user 201. The level of perceptibility may be deduced based on clinic-specific and/or user-specific data. Reference perceptibility value 371 may be clinic-specific and/or user-specific. Based on the comparing, it may be decided if one or more visualization parameters 341 of visual encoding scheme 350 are to be modified. The decision may be carried out by system 100. Based on the decision, the perceptibility of medical visualization 361 may be controlled by modifying 380 the one or more visualization parameters 341 of visual encoding scheme 361. If the decision is positive, the decision may comprise that one or more visualization parameters 341 of visual encoding scheme 350 are to be modified, and modifying 380 may comprise modifying the one or more visualization parameters 341. If the decision is negative, the decision may comprise that one or more visualization parameters 341 of visual encoding scheme 350 are not to be modified, and modifying 380 may comprise not modifying the one or more visualization parameters 341. Modifying 380 may cause rendering part 330 to adjust medical visualization 361 accordingly, so in accordance with the decision. Modifying 380 may comprise modifying the visualization parameter 341 of which the perceptibility is compared to reference perceptibility value 371: e.g., a heart symbol may be modified in size to be made larger than how the heart symbol is mapped via the mapping defined by visual encoding scheme 350, if the size of the heart symbol is smaller than the reference perceptibility value of the visualization parameter 341 `size', in order to make the heart symbol more noticeable in size. Alternatively, modifying 380 may comprise modifying one or more visualization parameters 341 which are different from the visualization parameter 341 of which the perceptibility is compared to reference perceptibility value 371: e.g., in the case of the same heart symbol its contours, a color in a used color palette, shape etc. may be made more noticeable to user 201 in various ways. For example, discreteness, contrast, glow, etc. may be added if it is found via the comparing to reference perceptibility value 371 that the perceptibility of the medical situation is too low, the noticeability of the medical situation to a human perception system, e.g., specifically of user 201, is too low, user 201 is likely to not notice the medical situation, and therefore has the overall perceptibility has to be increased; and fluency, smoothness, etc. may be added if it is found via the comparing to reference perceptibility value 371 that the perceptibility of the medical situation is too high, the noticeability of the medical situation to a human perception system, e.g., specifically of user 201, is too high, user 201 is likely to be unnecessarily triggered and/or distracted, and therefore the overall perceptibility has to be decreased. The one or more visualization parameters 341 which may be adapted may be comprised in a visualization pattern, jointly comprising one or more visualization parameters 341, in the visual encoding scheme 350. With such graphical perception compensation techniques, controllable noticeability of medical situations may be reached, in which system 100 may control which visualization parameters 341 should be noticed by user 201, dependent on the medical situation, independently of the used visual coding system in visual encoding scheme 350. Perceptive qualities, such as perceptibility and noticeability to a human perception system, and qualities, e.g. semantic qualities, as used in the graphical encoding of visual encoding scheme 350, is decoupled this way, by which the controllable aspect of the noticeability takes place in an independent manner. This way, independent control of the peripheral visibility of medical data may be achieved.

Visualization parameters 341 may vary in time. Modifying 380 may be synchronous with the varying in time of visualization parameters 341 which are modified. System 100 may be configured to add a visual and/or an auditory signal to medical visualization 361, such as a symbol, e.g., an alarming symbol, and/or a sound, e.g., an alarming sound. Adding a signal by system 100 may be based on the comparing and/or the decision. Modifying 380 may comprise a change in an animation in the medical visualization 361. Modifying 380 may comprise a change in a fluency of an animation in medical visualization 361. Modifying 380 may comprise an increase or decrease in gradation between values of one or more of visualization parameters 341 in an animation in medical visualization 361.

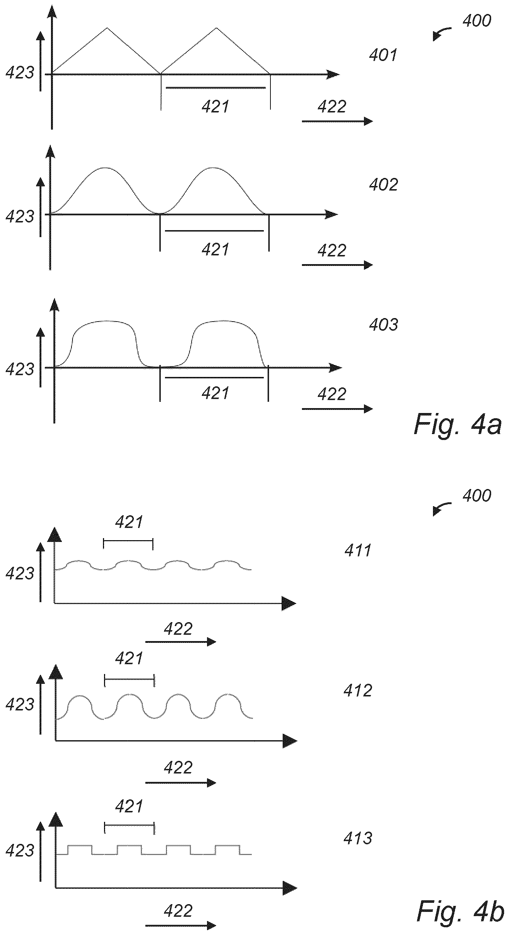

**Figs. 4a and 4b** schematically show examples of medical visualizations 400 wherein perceptibility is controlled according to an example of an embodiment; namely, in making animations less fluent, thereby increasing their noticeability. Medical visualizations 400 of, e.g., a periodic bodily function, such as, e.g., a heart rate, comprise visual representations 401, 402, 403 in **Fig. 4a****,** together with corresponding visual representations 411, 412, 413 in **Fig. 4b****.** Visual representations 401, 402, 403, 411, 412, 413 are all shown and plotted in apparent size 423 over time 422. As apparent size 423 changes over time 422 with a repetition rate 421, animations may be produced of visual representations 401, 402, 403. For example, visual representation 401 may then produce an animation with a difference in apparent size 423 of, e.g., 10%, displayed at a certain given repetition rate 423 and frequency. The sizes in between the minimum and maximum size, the amplitude, may be shown as fluently as possible, e.g., following a perfect sinus wave, as may be shown in corresponding visual representation 411. As another example, visual representation 402 may produce an animation with a same repetition rate 421 and frequency as for visual representation 401. However, the animation for visual representation 402 may generally be easier to notice for the human perception system of a user 201, as it uses a larger amplitude of difference in size 423, e.g., 20%, between the minimum and maximum size 423; as may be shown in corresponding visual representation 412. As a third example, visual representation 403 again uses the same repetition rate 421 and frequency as in visual representation 401. Visual representation 403 also uses a same amplitude of, e.g., 10% of difference in size 423; see corresponding visual representation 413. However, for visual representation 403 the produced animation may only animate between the two extreme states only, which may result in discrete or more discrete jumps in the resulting animation. In the resulting animation, the interpolated in-between states are not or less shown, unlike in the animation produced from visual representation 401. The resulting block-like pattern for visual representation 403 may generally have a higher noticeability for the human perception system than the visual representation of 401 and its resulting animation. Particularly the last discussed visual representation 403 may demonstrate that the perceptibility of a visual coding as in a visual encoding scheme 350, which in this case may be that of a heart-rate frequency, may be altered without 'touching' the visual coding, e.g., a semantic coding, itself: so, independently of the visual encoding scheme 350, as the fluency of the animation, which is a different visualization parameter from the size parameter, is decreased without this being encoded. The difference as seen in visual representations 411 and 412, so by changing the size difference, also demonstrates an altering independently from the visual encoding, as the size is altered beyond the encoding of the visualization parameter of size. Here, the same visualization parameter is altered. Such mechanisms may be applied in two directions, so to make a visual representation, such as an animation, more or less noticeable. This may depend on a desired noticeability in peripheral vision.

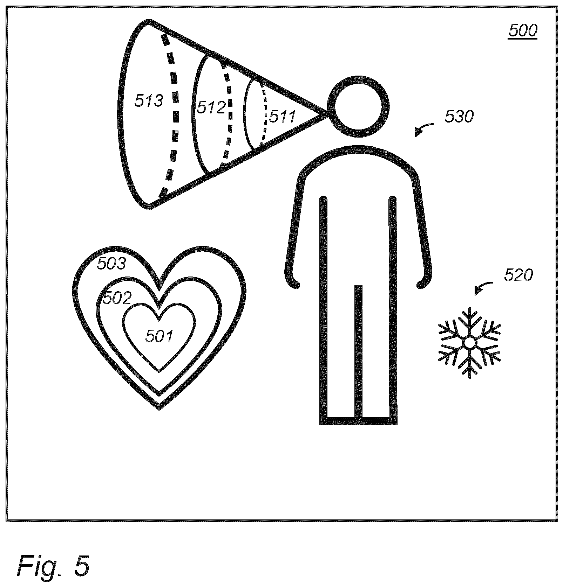

**Fig. 5** schematically shows a medical visualization 500 of which perceptibility is controlled according to an example of an embodiment. Medical visualization 500 may comprise a visual patient avatar. Medical visualization may comprise a symbol 530 of a body denoting a patient 205. Medical visualization 500 may comprise a variety of different bodily functions, such as, e.g., heart rate and respiration. In medical visualization 500, different stages of apparent sizes 501, 502, 503 of a medical visualization of a heart may be shown, in the form of heart symbols of growing sizes. In medical visualization 500, different stages of apparent sizes 511, 512, 513 of a medical visualization of an exhaling may be shown, in the form of cone-like shapes of growing sizes, starting from the mouth or head of symbol 530 denoting patient 205. In medical visualization 500, an ice flake symbol 520 indicating a low body temperature may be shown.

The coding of physiological parameters, e.g., measured physical parameters of patient 205, may use various graphical dimensions and combinations thereof as visualization parameters 341: e.g., one or more of color, shape, size, transparency, brightness, orientation, contrast, gradient, glow, fluency of an animation in the medical visualization, a repetition frequency of the medical visualization, a difference between visualization parameters, a variation in time of a visualization parameter, and/or a derivative or slope of a visualization parameter. For example, color may be used as a visualization parameter 341 to encode oxygen saturation, e.g., to let body symbol 530 turn purple in the case of a drop in oxygen saturation. One or more of graphical dimensions which may be used as visualization parameters 341 may be animated. This animation may be animated synchronously with corresponding physiological parameters, such as measured physical parameters, e.g., with a frequency which is in synchrony with a measured physical parameter. Frequency may also be used in the encoding, e.g., as an additional visualization parameter 341. For example, heart rate may be encoded with an animation of a heart symbol 501, 502, 503. The heart symbol may change in time, e.g., according to the different stages as shown in 501, 502, 503. The heart symbol may change slightly in size. The change in size of the heart symbol may happen synchronously with the measured heart rate as a physiological parameter. A single medical visualization may comprise a plurality of encodings in various visual representations. For example, a volume of carbon dioxide may be a further physiological parameter to be encoded. A corresponding visualization may be an animation of a cone-like shape symbol, 511, 512, 513, or a balloon-like symbol, which may grow in size, as if a balloon may be filled with air. The symbol may change in time, e.g., according to the different stages as shown in 511, 512, 513. The animation of a growing cone may be synchronous with the breathing rate of patient 205. The size of the cone-like shaped symbol 511, 512, 513 may represent the volume of carbon dioxide. Dependent on which graphical dimensions may be used as visualization parameters in the coding, further graphical dimensions may be used to increase or decrease the noticeability of a specific coding.

A possible measured physical parameter which may be used as a physiological parameter 321 in the coding may be a heart rate. Heart rate may be coded as follows. The frequency of the heart rate may be synchronous with the frequency of an animation. The animation may comprise a symbol of a heart 501, 502, 503. The heart symbol 501, 502, 503 may change in size 501, 502, 503 in the animation. There may be a size difference between the smallest size 501 and the largest size 503. The size difference may be, e.g., 10%. In order to increase the noticeability of a medical visualization of a heart rate, the size difference may be increased, e.g., from 10% to 20%; the animation may be rendered more jerky, so using less fluency, resulting in a block-like pattern in the animation, as the animation may transition between separate, distinct states, not showing any intermediate state; an overshoot in the largest and/or maximal size 503 may be added, e.g., a short overshoot of 5%; brightness, e.g., a high brightness blink, may be added when the heart symbol may reach a larger and/or maximal size 503; the heart symbol may vanish from medical visualization 500, as if it may be hid, between lower states, e.g., smaller sizes 501, and higher states, e.g., larger sizes 503, as if it may appear as 'blinking'; brightness may be added, e.g., the heart symbol may be rendered brighter in higher states, e.g., larger sizes 503, and/or the heart symbol may be rendered darker in lower states, e.g., smaller sizes 501; glow, e.g., a dark glow, may be added around the heart symbol in order to render the heart symbol more noticeable in with respect to the remainder of medical visualization 500. In order to decrease the noticeability of a medical visualization of a heart rate, smoothness, e.g., fluency of an animation, may be added, e.g. by rendering the animation of a heart rate in a smoother manner, e.g. following a sinus pattern; a color difference may be decreased or removed, e.g., a color difference between lower states, e.g., smaller sizes 501, and higher states, e.g., larger sizes 503; transparency may be added, e.g., via an increase of transparency in order to render the animation of heart rate less noticeable with respect to the remainder of medical visualization 500.

A possible measured physical parameter which may be used as a physiological parameter 321 in the coding may be expiratory carbon dioxide. Expiratory carbon dioxide may be coded as follows. A frequency of respiration or breathing rate may be synchronous with the frequency of an animation. The animation may comprise a cone-like or balloon-like symbol 511, 512, 513. The cone-like symbol 511, 512, 513 may change in size 511, 512, 513, e.g., denoting an exhale size, in the animation. There may be a size difference between the smallest size 511 and the largest size 513. The size difference may be, e.g., 10%. In order to increase the noticeability of a medical visualization of expiratory carbon dioxide, the exhale volume may be rendered in the visualization with more contrast to the background of medical visualization 500, e.g., by adding brightness, to render it less transparent, to add more glow around it; contrast may be let vary more in synchrony with the breathing rate frequency, e.g., by adding brightness in a higher state, e.g., a larger size 513 in the animation; the animation may be rendered jerkier, analogously to what has been discussed above. In order to decrease the noticeability of a medical visualization of expiratory carbon dioxide, smoothness, e.g., fluency of an animation, may be added, e.g. by rendering the animation in a smoother manner, e.g. following a sinus pattern; a smear effect and/or blurring effect may be added to the animation; contrast may be reduced, e.g., via reducing brightness; a transparency in the animation may be increased.

A possible measured physical parameter which may be used as a physiological parameter 321 in the coding may be oxygen saturation. Oxygen saturation may be coded as follows. A body symbol 530 denoting a patient 205 may change in color when there is a saturation drop, e.g., by the patient symbol 530 turning from, e.g., skin color, to purple. In order to increase the noticeability of a medical visualization of oxygen saturation, a difference in color from, a natural state color, e.g., skin color, to a lower saturation state, e.g., the color purple when there is a saturation drop, may be increased; a glow may be added; a brightness or color saturation changing animation may be added, wherein a higher frequency may be added to render it more noticeable. In order to decrease the noticeability of a medical visualization of oxygen saturation, a difference from the natural state color, e.g., skin color, to a saturation state color, e.g., purple, may be reduced via a decreased color saturation; a transparency of the color purple may be increased on top of the natural state color, e.eg., skin color.

A possible measured physical parameter which may be used as a physiological parameter 321 in the coding may be body temperature. A body temperature may be coded as follows. A body symbol 530 denoting a patient 205 may change in color when there is higher or lower body temperate, e.g., by turning blue in the case of a lower temperature or red in the case of a higher temperature. Body temperature may also be coded by adding a symbol, e.g., ice flake symbol 520, e.g. a blue ice flake symbol, in the case of a low body temperature of patient 205, and/or a corresponding symbol, e.g. temperature wave symbol, e.g. a heat wave symbol, e.g. an orange heat wave symbol, in the case of a high body temperature of patient 205. In order to increase the noticeability of a medical visualization of body temperature, a size a contrast, a color saturation and/or a brightness of the temperature symbols may be increased; an animation of the symbols may be added, e.g., varying in brightness, in size, in rotation, and/or in shape. In order to decrease the noticeability of a medical visualization of body temperature, a size, a contrast, a color saturation and/or a brightness of the symbols may be decreased; a transparency may be increased; the temperature symbols may be ensured to remain static, e.g., by not rendering them in an animated manner.

As discussed above, the coding of physiological parameters 321 using visualization parameters 341 in visual coding scheme 350 may be chosen with care, e.g., such that it may link well to the semantics and the mental model of medical professional users 201. This may generally lead to a well-comprehensible and well-interpretable coding for users 201. Increasing or decreasing the noticeability, e.g., using the options discussed above, may be desirable if the coding may go at risk of important medical situations not getting noticed, and/or unimportant medical situations attracting too much attention. Via, e.g., the means discussed above, this may be compensated for, and/or even overcompensated if desirable. The levels, e.g. various levels of smoothness of animations, at which the increasing or decreasing of the noticeability may be applied, e.g., using available graphical mechanics, may be varied in order to reach the right level of noticeability. When applying means, e.g. graphical mechanics, to increase or decrease noticeability of medical visualizations 361, 500, it may be important to understand that to some users 201, the used mechanisms might erroneously be interpreted as being part of an encoding, e.g., an extra encoding, of visual encoding scheme 350, instead of being an independent mechanism. For example, enlarging a size difference of a heart rate animation might make some users 201 think that the larger size 503 or smaller size 501 may show a corresponding pumping volume of the heart. Changing a different visualization parameter than size, such as animation frequency, may decrease such misinterpretation. Furthermore, user testing may be desirable in order to identify the `most correct' mechanics to use, as well as the right intensity with which one may apply them in order to get the desired effect in noticeability. The core encoding in visual encoding scheme 350 may have a certain noticeability at different levels, which may be considered as a baseline. The addition of graphical mechanics to compensate for these may be designed in such a way that the noticeability remains the same at all levels of the original encoding in visual encoding scheme 350. The used graphical mechanics may be applied to increase or decrease the noticeability based on the clinical situation and/or the medical situation of a patient 205, and thus the required attention, at hand.

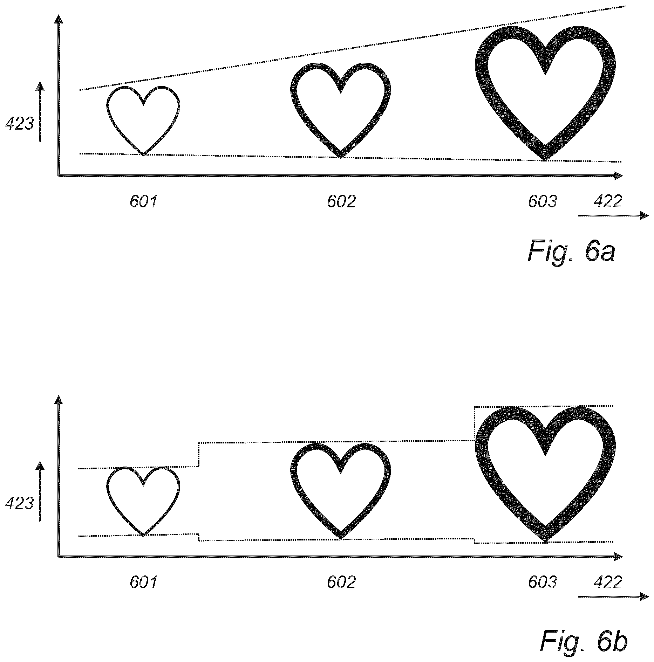

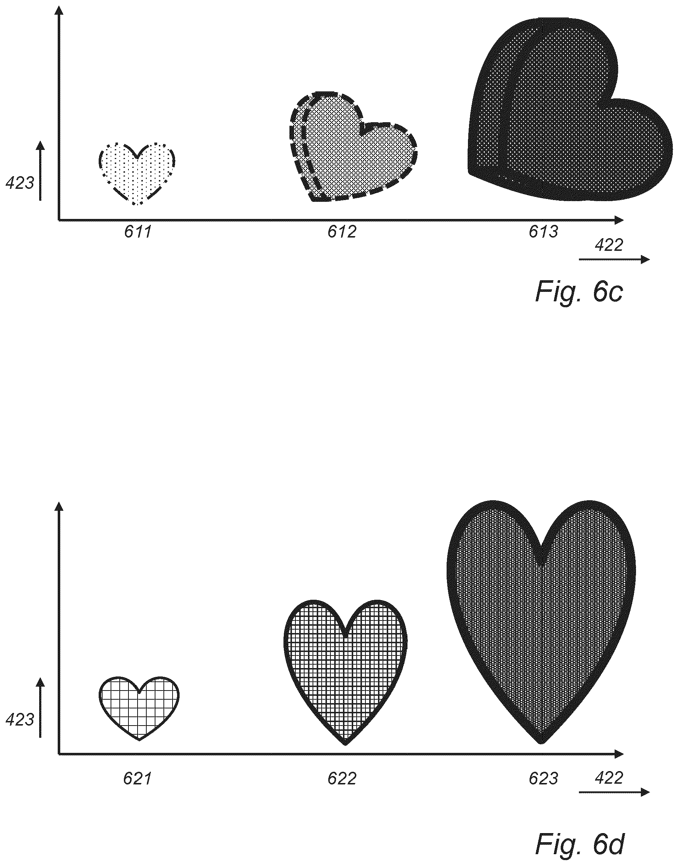

**Figs. 6a, 6b****,** **6c and 6d** schematically show examples of medical visualizations 601, 602, 603, 611, 612, 613, 623, 622, 623 of a heart, of which perceptibility is controlled according to an example of an embodiment. In these examples, apparent size 423 may be plotted with respect to time 422.

Medical visualizations 601, 602, 603 in Fig. 6a and 611, 612, 613 in Fig. 6b may be seen visualizations to be animated. Over time 422, both size and contour thickness increase. Next to this, the visualization in a possible animation may employ higher frequency rates, at which the heart symbol may change in size and contour thickness. Higher rates, visualized in high-frequency animations, typically correlate with a higher noticeability; lower rates, typically correlate with lower noticeability. This relationship may however be decoupled, by reducing fluency of the animation, resulting in more jerkiness, or discreteness, in slower animations; more fluent animations may be used in faster animations to compensate for this. In Figs. 6a and 6b, the dashed lines may denote fluencies of the animation. The dashed line in Fig. 6b may indicate a more discrete or jerky and less fluent animation, by showing fewer stages or frames of heart size and contour thickness. Thereby, this animation may be made more noticeable.

In Figs. 6c and 6d, more examples are shown of possible visualization parameters which may be modified in order to make a visualization, e.g., an animation, more noticeable or less noticeable. In Fig. 6c, the heart symbol increases in size, in contour thickness, and in 'tiltedness' from an equilibrium state. Note that varying the tiltedness may confuse a medical professional on what is going on with the patient, and another visualization parameter may be a more suitable choice to be modified for adapting perceptibility of heart rate. Moreover, the heart symbol is shown in a more 3-D like manner, as if it stands out of the 2-D figure; and the dashing pattern of the contour of the shape as well as the dash-fill pattern of the shape itself of the symbol changes over time. In Fig. 6d, the heart symbol increases in size and contour thickness. Moreover, over time the heart symbol is shown in a more in elongated manner in its shape from an equilibrium state. Furthermore, the filling pattern of the shape is changed from a wide block pattern via a narrow block pattern to a narrow dot pattern, and the density of this pattern is increased.

Visualization parameters may additionally comprise a difference between visualization parameters, a variation in time of a visualization parameter, and/or a derivative or slope of a visualization parameter. A derivative of a change may additionally be used as a visualization parameter on its own. For a human perception system, first and second parameters may be most important in rendering an animation. Other derivatives, from other visualization parameters, may also be derived and used as visualization parameters on their own. For example, an importance of a change in a visualization parameter may be additionally used as a visualization parameter. Such visualization parameter may be used in controlling the perception of a visualization, and may improve, instead of controlling, the comprehension of a user 201. A visualization parameter such as importance may also change of over time. By this, also its change, derivative, and variation over time may be used as visualization parameters.

A visualization, such as an animation, may be perceived and noticed as more aggressive if it comprises larger amplitudes in its derivatives. This may obtain more noticeability and therefore more attraction from a user 201. See also visualization 403 in Fig. 4a and the corresponding visualization 413 showing the differences in Fig. 4b. This aggression of a visualization may be reduced after some time in which it may be assumed that attraction from a user 201 may be obtained. Then the situation of the animation may be reduced to a visualization of the sort of 402 in Fig. 4a and the corresponding visualization in 412 showing the differences in Fig. 4b. Note that the rate is still the same, as the medical situation is still the same, but the situation attracts less attraction according to the human perception system, which means that this situation will less likely overblow other, possible important, changes in the medical situation in the peripheral view 220.

The jerkiness and thus the noticeability of an animation may also be further amplified via a mechanism as follows. A difference in a visualization parameter serving as a signal, e.g., a brightness between two frames in an animation, may be defined as a slope. The slope is a first derivative of the brightness signal and may be perceived as a parameter such as smoothness. The derivative may be a calculatable value of smoothness. Note that smoothness is an observable parameter value for the human perception system, by which the derivative is a perceivable visualization parameter in its own. The derivative may then be what is noticed in peripheral view by the human brain of a user 201. The derivative, and/or a second derivative, may be modified as a visualization to get smoother slopes of other visualization parameters. A slope may be increased to obtain a more aggressive effect in an animation, similar to what LCD displays in, e.g., televisions may do to get 'faster switching', which may be similar to what we want to achieve with a lower noticeability (smoother motion attracts less attention). Furthermore, a higher slope may mean in signal theory that a higher frequency in a signal may be needed. For example, if it is desirable to go from a lower state of color, e.g., a dark grey value, to a higher state of color, e.g., a light gray value, one may insert a black and a white image as a sort of slope between the two states. To go from dark grey as a color to a black image, to a white image, and then a light gray image may be called using an overshoot, or an undershoot. For other colors, using an overshoot or undershoot may work similarly. Physical aspects may limit such a usage in the case of colors. Using a higher slope than the slope from the color black to the color white within a short timeframe, e.g., 16ms, may not be possible. Using such an overshoot may work for movements in animations a well: if an image may go into an incorrect direction, a next image may be rendered as going further away than necessarily may be required, by which a third image may be shown in a final, correct position. This may also create the idea of a faster movement to a human perception system. Slope itself may be used as a visualization parameter, and it may be read by the human perception as a perceivable parameter on its own, by which it may attract attention of a user 201 on its own, as the resulting parameter may control the attention a change in the visualization parameter of which the slope is considered may get from a user 201. In an analogous way, the slope may attract attention away, and reduce the attention a visualization may get. Thereby, a situation of visual fatigue of the human brain of a user may be avoided. Similarly, as for slopes, first, second and higher derivatives of visualization parameters may be used as new parameters.

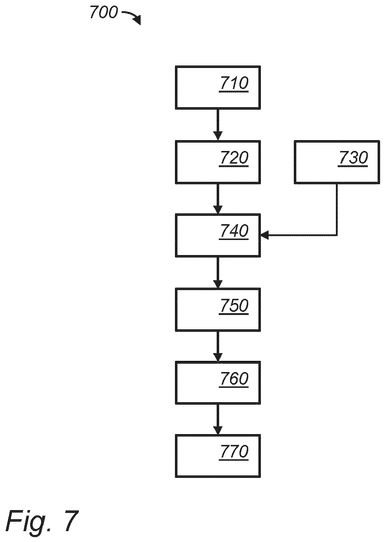

**Fig. 7** schematically shows an example of an embodiment of a method 700 of controlling perceptibility of a medical visualization 361. Method 700 may comprise obtaining 710 physiological data 321 of a patient 205. Method 700 may comprise mapping 720 physiological data 321 to medical visualization 361, wherein mapping 720 may be defined by visualization parameters 341 comprised in a visual encoding scheme 350. Method 700 may comprise selecting 730 a visualization parameter 341 of which the perceptibility is to be compared. Method 700 may comprise comparing 740 the perceptibility of visualization parameter 341 to a reference perceptibility value 371, wherein reference perceptibility value 371 indicates a level of perceptibility to a human perception system of a user 201. Method 700 may comprise deciding 750, based on the comparing 740, if one or more visualization parameters 341 of visual encoding scheme 350 are to be modified. Method 700 may comprise controlling 760, based on the deciding 750, the perceptibility of medical visualization 361 by modifying 380 the one or more visualization parameters 341 of visual encoding scheme 350. Method 700 may comprise adding 770 a visual and/or an auditory signal to medical visualization 361.

Many ways of executing method 700 are possible, as will be apparent to a person skilled in the art. For example, the order of the steps can be performed in the shown order, but the order of the steps can be varied, or some steps may be executed in parallel. Moreover, in between steps other method steps may be inserted. The inserted steps may represent refinements of method 700 such as described herein or may be unrelated to method 700. For example, some steps may be executed, at least partially, in parallel. Moreover, a given step may not have finished completely before a next step is started.

Embodiments of method 700 may be executed using software, which comprises instructions for causing a processor system to perform method 700. Software may only include those steps taken by a particular sub-entity of the system. The software may be stored in a suitable storage medium, such as a hard disk, a memory, an optical disc, etc. The software may be sent as a signal along a wire, or wireless, or using a data network, e.g., the Internet. The software may be made available for download and/or for remote usage on a server. Embodiments of method 700 may be executed using a bitstream arranged to configure programmable logic, e.g., a field-programmable gate array, to perform method 700.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

Method 700 may be a computer-implemented method. For example, accessing and sharing the training data, and/or receiving other input data may be done using a communication interface, e.g., an electronic interface, a network interface, a memory interface, etc. For example, storing or retrieving training parameters may be done from an electronic storage, e.g., a memory, a hard drive, etc. For example, adjusting stored parameters may be done using an electronic computing device, e.g., a computer.

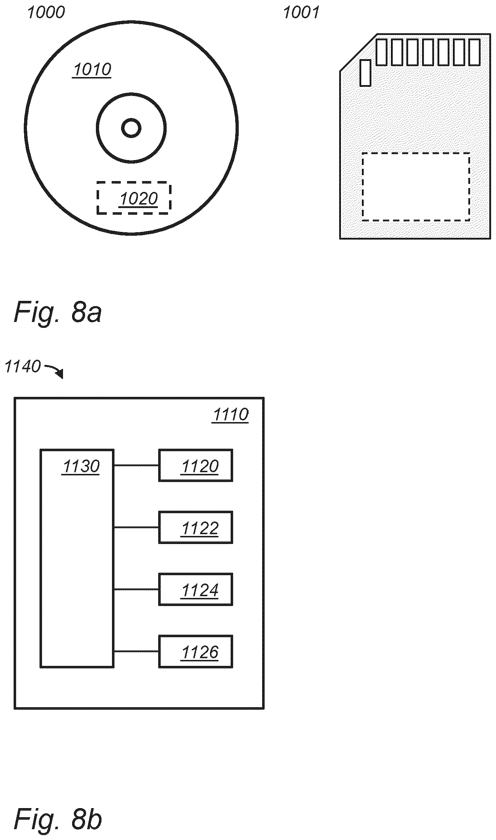

**Fig. 8a** schematically shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of method 700. Data 1020 may comprise a computer program 1020 according to an embodiment, The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 may comprises instructions for causing a processor system to perform an embodiment of method 700.

**Fig. 8b** schematically shows a schematic representation of a processor system 1140 according to an embodiment. The processor system comprises one or more integrated circuits 1110. The architecture of the one or more integrated circuits 1110 is schematically shown. Circuit 1110 comprises a processing unit 1120, e.g., a CPU, for running computer program components to execute a method 700 according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140 may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While system 1140 is shown as including one of each described component, the various components may be duplicated in various embodiments. For example, the processing unit 1120 may include multiple microprocessors that are configured to independently execute the methods described herein or are configured to perform steps or subroutines of the methods described herein such that the multiple processors cooperate to achieve the functionality described herein. Further, where the system 1140 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A system (100) for controlling perceptibility of a medical visualization (361), the system (100) comprising a processor system (110) and a memory (120) storing instructions that, when executed by the processor system (110), cause the processor system (110) to perform operations for:
- a rendering part (330), wherein the rendering part (330) maps (720) physiological data (321) of a patient (205) to a medical visualization (361), wherein the mapping (720) is defined by visualization parameters (341) comprised in a visual encoding scheme (350),
- comparing (740) a perceptibility of a visualization parameter (341) of the visual encoding scheme (350) to a reference perceptibility value (371), wherein the reference perceptibility value (371) indicates a level of perceptibility to a human perception system of a user (201),
- based on the comparing (740), deciding (750) if one or more visualization parameters (341) of the visual encoding scheme (350) are to be modified (380), and
- based on the deciding (750), controlling (760) the perceptibility of the medical visualization (361) by modifying (380) the one or more visualization parameters (341) of the visual encoding scheme, the modifying (380) causing the rendering part (330) to adjust the medical visualization accordingly.

2. A system (100) according to Claim 1, wherein the modifying (380) comprises modifying the visualization parameter (341) of which the perceptibility is compared (740) to the reference perceptibility value (371).

3. A system (100) according to any of the preceding claims, wherein the modifying (380) comprises modifying one or more visualization parameters (341) which are different from the visualization parameter (341) of which the perceptibility is compared (740) to the reference perceptibility value (371).

4. A system (100) according to any of the preceding claims, wherein the visualization parameters (341) vary in time, and the modifying (380) is synchronous with the varying in time of the one or more visualization parameters (341) which are modified.

5. A system (100) according to any of the preceding claims, wherein the system (100) is configured to:
- based on the comparing (740), add (770) a visual and/or an auditory signal to the medical visualization (361), such as a symbol and/or a sound.

6. A system (100) according to any of the preceding claims, wherein the visualization parameters (341) comprise one or more of: color, shape, size, transparency, brightness, orientation, contrast, gradient, glow, fluency of an animation in the medical visualization (361), and a repetition frequency of the medical visualization (361).

7. A system (100) according to any of the preceding claims, wherein the visualization parameters (341) additionally comprise a difference between visualization parameters (341), a variation in time of a visualization parameter (341), and/or a derivative or slope of a visualization parameter (341).

8. A system (100) according to any of the preceding claims, wherein the modifying (380) comprises a change in a fluency of an animation in the medical visualization (361).

9. A system (100) according to any of the preceding claims, wherein the modifying (380) comprises an increase or decrease in gradation between values of a visualization parameter (341) in an animation in the medical visualization (361).

10. A system (100) according to any of the preceding claims, wherein the visual encoding scheme (350) comprises one or more visualization patterns, the one or more visualization patterns comprising the visualization parameters (341).

11. A system (100) according to any of the preceding claims, wherein the system (100) is configured to select (730) the visualization parameter (341) of which the perceptibility is compared (740).

12. A system (100) according to Claim 11, wherein the selecting (730) is based on values of the physiological data (321) of the patient (205).

13. A system (100) according to any of the preceding claims, wherein the system (100) is configured to enable the user (201) to select (320) the visualization parameter (341) of which the perceptibility is compared (740).

14. A system (100) according to any of the preceding claims, wherein the system (100) is further configured to obtain (710) the physiological data (321) of the patient (205) from a sensor (310).

15. A method (700) of controlling perceptibility of a medical visualization (361), comprising:
- mapping (720) physiological data (321) of a patient (205) to a medical visualization (361), wherein the mapping (720) is defined by visualization parameters (341) comprised in a visual encoding scheme (350),
- comparing (740) a perceptibility of a visualization parameter (341) of the visual encoding scheme (350) to a reference perceptibility value (371), wherein the reference perceptibility value (371) indicates a level of perceptibility to a human perception system of a user (201),
- based on the comparing (740), deciding (750) if one or more visualization parameters (341) of the visual encoding scheme (350) are to be modified (380), and
- based on the deciding (750), controlling (760) perceptibility of the medical visualization (361) by modifying (380) the one or more visualization parameters (341) of the visual encoding scheme (350).
